# EUROPEAN PATENT SPECIFICATION

(11) **EP 3 860 693 B1**
(45) Date of publication and mention of the grant of the patent: **10.04.2024**
(21) Application number: 19780413.1
(22) Date of filing: 20.09.2019
(51) Int. Cl.: A61M 5/315, A61M 5/24, A61M 5/28, A61M 5/19

(54) **SYRINGE FOR INJECTING AN INJECTABLE SOLUTION CONTAINED IN A DEFORMABLE CARTRIDGE**
SPRITZE ZUM EINSPRITZEN EINER INJIZIERBAREN LÖSUNG, DIE IN EINER VERFORMBAREN KARTUSCHE ENTHALTEN IST
SERINGUE POUR INJECTER UNE SOLUTION INJECTABLE CONTENUE DANS UNE CARTOUCHE DÉFORMABLE

(30) Priority: 03.10.2018 IT 201800009134
(43) Date of publication of application: 11.08.2021
(73) Proprietor: Orofino Pharmaceuticals Group SRL, 00193 Roma (IT)
(72) Inventor: OROFINO, Ernesto, 00193 Roma (IT)
(74) Representative: Carangelo, Pierluigi
(86) International application number: PCT/IB2019/057978
(87) International publication number: WO 2020/070577

(56) References cited:
- WO-A1-2017/127287
- WO-A2-2011/109840
- US-A1- 2001 047 162

## Description

The present invention relates to the technical field of injection devices and in particular it relates to a syringe for injecting an injectable solution contained in a deformable cartridge.

Currently, sterile raw materials in powder form are mainly packed by active ingredient manufacturers either in aluminum containers or in plastic bags, generally made of polyethylene.

In order to be able to market them, manufacturers have demonstrated that each raw material, in addition to maintaining sterility, remains stable in the containers or bags used for a given period of time; in other words, such powders do not decay for a predetermined number of years when stored in such containers or bags.

The sterile raw materials are sold to finished pharmaceutical product manufacturers, who fractionate them using known technologies, marketing the finished product in sterile powder form contained in a bottle. The bottle is accompanied by a glass vial containing the solvent.

The use of high-quality materials for the vial (glass), of compatible materials for the sterile powder and of disposable syringes make this system very costly as a whole.

The use of syringes provided with a compartment for receiving a deformable cartridge made of flexible material containing pharmaceutical substances kept separate in respective containing chambers by a special soft partition until the moment of use is known in the sector. An example of such devices is described in patent US2001/0047162. A further example of such known devices is described in patent WO2017137854 A1. WO 2011/109840 A2 discloses a linearly actuated dispenser and therapeutic package suitable for administering a therapeutic substance.

However, it has been observed that the operations of inserting the deformable cartridge into the syringe in the syringes of the prior art are relatively complex. Furthermore, if the insertion is not performed correctly, the cartridge may be damaged and/or the injection may not be performed correctly.

It is the object of the present invention to solve the problems of the syringes for deformable cartridges of the prior art described above. In particular, it is the object of the present invention to provide a syringe for injecting an injectable solution contained in a deformable cartridge which allows an easy and precise insertion of the cartridge into the syringe itself.

Such an object is achieved by a syringe for injecting an injectable solution contained in a deformable cartridge according to claim 1 and by an injection kit according to claim 9.

The features and advantages of the syringe according to the present invention will be apparent from the following description, given by way of non-limiting example, according to the accompanying drawings, in which:
- figure 1 shows a plan view from the top of a possible embodiment of a deformable cartridge;
- figure 2 shows a side plan view of the deformable cartridge in figure 1;
- figure 3 shows a side plan view of a separation device of the deformable cartridge in figure 1;
- figure 4 shows an axonometric side view of the deformable cartridge in figure 1;
- figure 5 shows a further axonometric view of the deformable cartridge in figure 1 in which two parts of the deformable cartridge detached from each other are shown;
- figure 6 shows an axonometric view of a kit comprising the cartridge in figure 1 and a syringe according to a possible non-limiting embodiment of the present invention, in which the syringe is shown in a first operating configuration;
- figure 7 shows a further side view of the kit in figure 6, in which the syringe is shown in the first operating configuration;
- figure 8 shows an axonometric view of the syringe in figure 6 alone, in which the syringe is shown in a second operating configuration;
- figure 9 shows an axonometric section view of part of the syringe in figure 6;

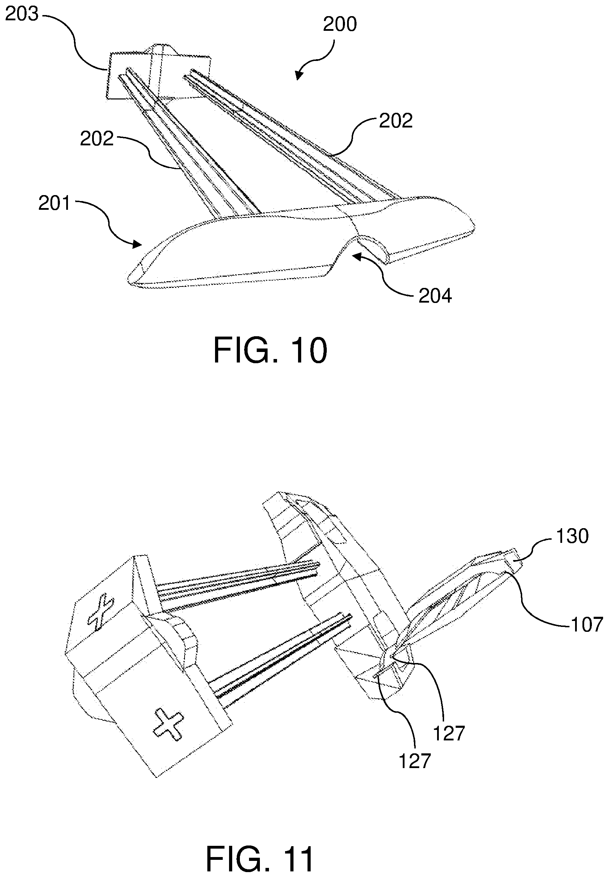

- figure 10 shows an axonometric view of a further part of the syringe in figure 6;
- figure 11 shows an axonometric view of the syringe in figure 6 from which the deformable cartridge was removed.

Similar or equivalent elements are indicated by means of the same reference numerals in the aforesaid figures.

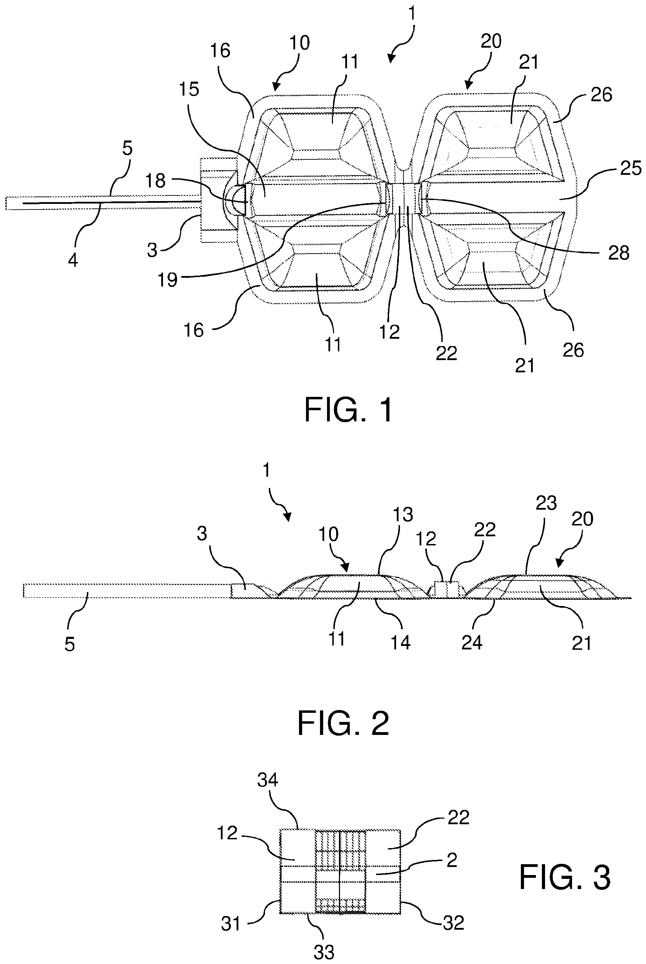

Before describing the syringe, we believe it is worth describing a non-limiting example of a deformable cartridge which can be used with such a syringe. In this regard, figures 1-5 show a non-limiting embodiment of a deformable cartridge 1 which can be used in a syringe according to the present invention. The deformable cartridge 1 has a cartridge body 10,20 comprising:
- at least one first containment chamber 11 of a first component of the injectable solution, in which the first containment chamber 11 comprises at least one first compression-deformable wall;
- at least one second containment chamber 21 of a second component of the injectable solution, in which the second containment chamber 21 comprises at least one second compression-deformable wall in order to allow the second component to be transferred from the second containment chamber 21 to the first containment chamber 11, whereby mixing the first component and the second component.

According to a particularly advantageous embodiment, the cartridge body 10,20 is a blister. In this case, the aforesaid compression-deformable walls are the walls of at least two blister pockets delimiting the first containment chamber 11 and the second containment chamber 21, respectively. According to an alternative embodiment, the cartridge body 10,20 comprises one or more bags with flexible walls.

The first containment chamber 11 and the second containment chamber 21 will also be indicated hereinafter in this description using the expressions front chamber 11 and rear chamber 21, respectively. It is worth noting that the structure may be varied also by providing more rear chambers 21.

According to an advantageous and non-limiting embodiment, the outer walls of the containment chambers 11,12 are at least partly coated with a protective adhesive film which can be removed before the injectable substance is reconstituted. Such a film allows for increased segregation between the containment chambers under storage conditions.

It is worth noting that the teachings of the present description also extend to deformable cartridges having a single containment chamber, i.e. to cartridges containing an injectable solution already pre-constituted at the factory. Hereinafter, however, reference will be made to the case in which the deformable cartridge comprises two containment chambers, without thereby introducing any limitation.

Each containment chamber 11,21 contains one of the components of the injectable solution; for example, the front chamber 11 contains a first component in the form of a sterile drug powder and rear chamber 21 contains a second component, which is a liquid component, typically a solvent or a further active pharmaceutical ingredient. For example, the sterile drug powder is a crystallized powder or a powder obtained in bulk by the liophilization of a solution. As an alternative to powder, the first component is a granular substance or a sterile tablet or a compacted powder. According to a possible embodiment, the first component comprises two distinct substances, e.g. in the form of two distinct tablets, each containing one of said two distinct substances.

The first component is, for example, a highly active substance, such as, for example: a beta-lactam antibiotic, such as a cephalosporin antibiotic, or a cytotoxic anticancer substance or a hormone or biological preparation. The aforesaid first component may also be a normal active substance, i.e. not definable as a highly active substance.

The aforesaid first component may also be a liquid component, as an embodiment of the deformable cartridge 1 can be envisaged in which both the first component and the second component are liquid substances.

Preferably, the second component is a solvent for injectable use, e.g. a WFI (Water For Injection) solvent or a lidocaine solution or a solution of water and benzyl alcohol or a saline solution of sodium chloride or generally any injectable substance capable of reconstituting another solid or liquid substance. The second component may either be or contain an API (Active Pharmaceutical Ingredient).

According to a particularly advantageous embodiment, the deformable cartridge 1 further comprises a separation device 12,22 operatively interposed between the first containment chamber 11 and the second containment chamber 12 having a device body comprising at least one fluidic communication duct 2 defined in the device body. The body of the device is preferably a stand alone body and preferably is more rigid than the deformable walls of the first containment chamber and of the second containment chamber. Such a device body is either inserted into or attached to the cartridge body 10,20, e.g. either inserted or engaged into the cartridge body 10,20, preferably inserted into a deformable blister-type cartridge. It is possible to provide a plurality of communication ducts 2.

Preferably, the deformable cartridge 1 is adapted to take a first operating configuration, in which the fluidic communication duct 2 of the separation device 12,22 cannot be crossed by the second component, and a second operating configuration, in which the fluidic communication duct 2 can be crossed by the second component. In other words, in the first operating configuration, the separation device 12,22 does not allow fluidic communication between the second chamber 21 and the first chamber 11, while in the second operating configuration the separator 12,22 allows fluidic communication between the second chamber 21 and the first chamber 11.

According to particularly advantageous embodiments, the separation device 12,22 comprises a filter or a valve or a connector. For example, the separation device 12,22 comprises a valve adapted to allow a unidirectional flow in the fluidic communication duct 2. Either additionally or alternatively, such a valve allows the flow of a liquid component into the fluidic communication duct 2 only if the pressure in the second chamber exceeds a threshold value. Valves of this type are known and used in reconstitution syringes having two or more chambers.

If the separation device 12,22 comprises a connector, such a connector may further comprise a filter and/or a valve integrated in the connector itself.

Preferably, the body of the separation device extends between a first face 31 facing towards the first containment chamber 11 and a second face 32 opposite to the first face 15 and facing towards the second containment chamber 21. More preferentially, the fluidic communication duct 2 is a recess or hole which extends from the first face 31 to the second face 32.

There are a third flat face 33 and a fourth curved and convex face 34 between the first face 31 and the second face 32 of the body of the separation device 12,22.

According to an advantageous embodiment, the fluidic communication duct 2 has a smaller cross-section than the size of the cross-section of the body of the separation device 12,22.

The deformable cartridge 1 further comprises a needle holder component 3 fixed to the cartridge body 10,20 and adapted to be configured to be fluidly connected to the first chamber 11. The first chamber 11 is operationally interposed, e.g. arranged, between the needle holder component 3 and the separation device 12,22. Preferably, the deformable cartridge 1 further comprises an injection needle 4 attached to the needle holder component 3 and a removable protective cap 5 mounted either on the injection needle 4 or on the needle holder component 3.

According to an advantageous embodiment, the cartridge body 10,20 comprises:
- a first sheet portion 13 and a second sheet portion 14 joined together, e.g. welded or glued, to form the first containment chamber 11;
- a third sheet portion 23 and a fourth sheet portion 24 joined together, e.g. welded or glued, to form the second containment chamber 21.

The sheet portions can be welded by applying heat, by applying radio frequency or ultrasonic energy or by means of any other appropriate welding technique.

For example, the first sheet portion 13 and the third sheet portion 23 are two portions of the same first sheet, e.g. of a multilayer film, the innermost layer of which is preferably a polyethylene layer, or of a single-ply polyethylene film. For example, the second sheet portion 14 and the fourth sheet portion 24 are also two portions of the same second sheet. The second sheet may also be a multi-layer film, the inner layer of which is preferably a polyethylene layer, or a single-layer polyethylene film. In such a manner, the cartridge body 10,20 is obtained, for example, by shaping the first sheet so as to create recesses at the containment chambers 11,21 and by sealing, e.g. by welding or gluing, the first and second sheets to each other outside such recesses on joining edges, which are peripheral edges of said sheets.

According to a particularly advantageous embodiment, the separation device 12,22, in particular the device body, is interposed between the first sheet portion 13 and the second sheet portion 14 and between the third sheet portion 23 and the fourth sheet portion 24. For example, the separation device 12,22 is interposed and fixed between the aforesaid first and the second sheet and is housed, for example, in a mutually counter-shaped recess defined in the aforesaid first sheet.

Preferably, the first sheet portion 13 and the third sheet portion 23 are relatively more flexible than the second sheet portion 14 and the fourth sheet portion 24 respectively, and carry the aforementioned deformable walls of the first containment chamber 11 and the second containment chamber 21, respectively.

According to an embodiment, the first sheet portion 13 and the third sheet portion 23 comprise at least one first and one second deformable rounded recess, respectively, below which the first containment chamber 11 and the second containment chamber 21 are arranged, respectively. In such an embodiment, the second sheet portion 14 and the fourth sheet portion 24 are flat and mutually coplanar, as shown in figure 2.

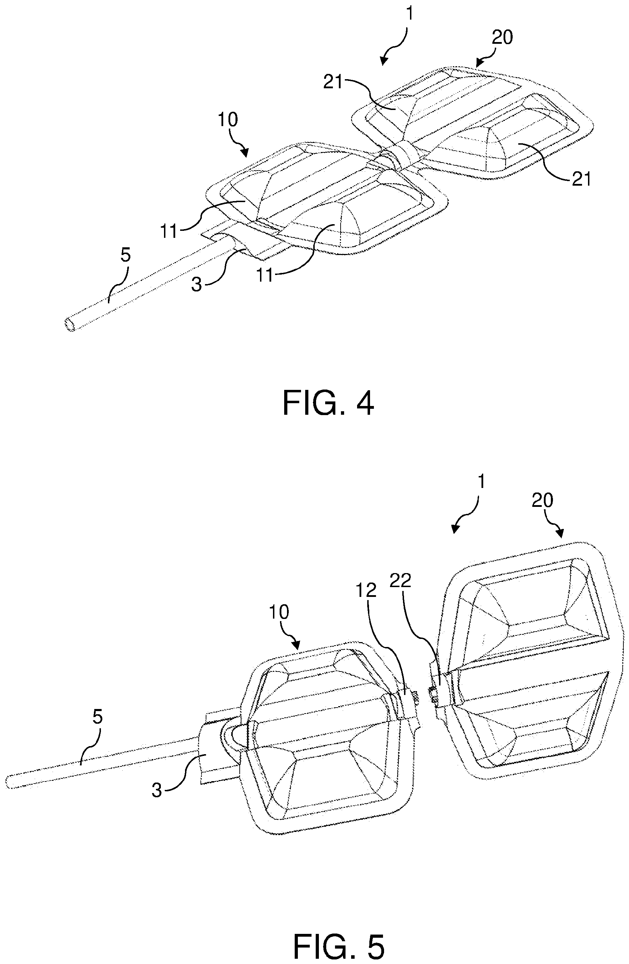

These considerations also apply to the case in which the first sheet portion 13 and the third sheet portion 23 are sheet portions which are mutually distinct and the second sheet portion 14 and the fourth sheet portion 24 are mutually distinct sheet portions. For the purposes of the present description, "separate sheets" means sheets which are not continuously joined to form a single continuous sheet and this definition thus also comprises sheets which are obtained from a single sheet but which have then been separated, e.g. cut, so that they no longer form a single continuous sheet. With reference to figure 4, in the aforesaid cases, the cartridge body 10,20 comprises two distinct parts 10, 20 which are or can be mechanically coupled by means of the separation device 12,22, which in this case comprises a connector. Such a connector is conveniently a fluid-tight connector. As explained above, such a connector may comprise a filter and/or a valve.

In particular, a particularly advantageous embodiment can be envisaged in which the separation device 12,22 comprises a connector with a first connection element 12 and a second connection element 22 and the cartridge body 10,20 comprises a first part 10 with the first connection element 12 and a second part 20 with the second connection element 22. The first connection element 12 and the second connection element 22 are mutually complementary and can be mechanically coupled together to form said connector and to fix the first part 10 and the second part 20 together to form said cartridge body 10,20.

In the aforesaid embodiment, in which the cartridge body 10,20 is formed by two detached parts, at least initially, for example, the needle holder component 3 is fixed to the first part 10 of the cartridge body 10,20.

In the embodiment in which the separation device 12,22 either is or comprises a connector, it is advantageous to envisage such connector as a connector with rotational closure. For example, the connection elements 12,22 may be joined to one another by means of a insertion-rotation-locking system, e.g. by means of an insertion-rotation-locking system with a quarter turn.

It is worth noting that it is not necessary for the connection elements 12,22 to be initially disconnected from each other because, for example, an embodiment can be envisaged in which such connection elements 12,22 are already engaged or constrained to one another and in which, for example, a rotation between such connection elements 12,22, e.g. equal to 180°, is such that it opens the fluidic communication duct 2, e.g. by either breaking or removing a barrier which initially occludes such a duct 2. In a further embodiment, it is possible to provide that two initially misaligned duct portions, respectively defined in the connection element 21 and in the connection element 22, are axially aligned by means of a rotation between the connection elements 12,22, so that the rotation allows the definition of a duct 22 capable of putting the second containment chamber 21 into communication with the first containment chamber 11.

According to a preferred embodiment, an outlet conduit 18 from the first chamber 11 closed by a peelable partition is defined between the first containment chamber 11 and the needle holder component 3. Such a peelable partition is obtained, for example, by a weak weld, e.g. a thin weld, between the first sheet portion 13 and the second sheet portion 14. Such a weld is configured to open when the pressure of the solution inside the first containment chamber 11 reaches a pressure value selected according to design specifications. An inlet duct 19 to the first containment chamber 11, closed by a peelable partition, is defined either additionally or alternatively, between the first containment chamber 11 and the separation device 12,22. Such a peelable partition is obtained, for example, by a weak weld, e.g. a thin weld, between the first sheet portion 13 and the second sheet portion 14. Such a weld is configured to yield when the pressure of the solution inside the second containment chamber 21 reaches a pressure value selected according to design specifications. Furthermore, either additionally or alternatively to the embodiments described in the present paragraph, an outlet conduit 28 from the second chamber 21, closed by a peelable partition, is defined also between the second containment chamber 21 and the separation device 12,22. Such a peelable partition is similar to those described above.

According to an advantageous embodiment, the first containment chamber 11 comprises two chamber portions 11 which are spatially separated from each other and arranged in parallel between the needle holder component 3 and the separation device 12,22. In an even more advantageous embodiment, the cartridge body 10,20 comprises a central part 15,25, two side parts 16,26 arranged on mutually opposite sides with respect to the central part 15,25. Furthermore, the separation device 12,22 is arranged on said central part 15,25 and the chamber portions 11 are arranged on the side parts 16,26, separated from each other by said central part which is a region glued or welded, for example. This advantageously allows the effective crushing and complete emptying of the two portions of chamber 11 by means of a crushing device 200 adapted and configured to pass over the separation device 12,22 without interfering with the latter.

Preferably, the second containment chamber 21 also comprises two chamber portions 21 which are spatially separated from each other and each of them is arranged on a respective side part 26. In such a manner, coherently with the embodiment shown in the accompanying figures, the cartridge body 10,20 is formed by a first part 10 and a second part 20, preferably but by way of non-limiting example initially mutually separated. The first part 10 comprises two side portions 16 on which the two portions 11 of the first containment chamber are housed separated from each other by a central part 15. Preferably, the two portions 11 of the first chamber have a trapezoidal or essentially trapezoidal plan shape. The second part 20 comprises two side portions 26 on which the two portions 21 of the second containment chamber are housed separated from each other by a central part 25. Preferably, the two portions 21 of the second containment chamber have a trapezoidal or substantially trapezoidal plan shape. The separation device 12,22 is interposed between the two central parts 15,25. According to an advantageous embodiment, the first part 10 and the second part 20 have a hexagonal shaped, or substantially hexagonal shaped, plan, e.g. a hexagonal shaped plan with rounded corners. The two parts 10,20 are joined at one side of the hexagon and the cartridge body 10,20 is substantially butterfly-shaped.

According to a possible embodiment, the two portions of chamber 11 of the first containment chamber 11 are fluidly communicating with each other, either from the beginning or after the breaking of one or more interposed peelable partitions. The same applies to the two chamber portions 21 of the second containment chamber 21.

However, in an embodiment which advantageously allows ensuring better control of the mixing between the first component and the second component:
- the two chamber portions 11 of the first containment chamber 11 are fluidically isolated from each other;
- the two chamber portions 21 of the second containment chamber 21 are fluidically isolated from each other;
- the separation device 12,22 comprises a first communication duct adapted and configured to fluidically interconnect only one of the two chamber portions of the second containment chamber 21 with only one of the two chamber portions 11 of the first containment chamber and a second communication duct adapted and configured to fluidically interconnect the other of the two chamber portions of the second containment chamber 21 only with the other of the two chamber portions of the first containment chamber 11.

In the last embodiment described above, the solution contained after mixing the two components in the two chamber portions 11 of the first containment chamber can be transferred to the needle 4 by providing a bifurcated fluidic duct, e.g. T-shaped or Y-shaped, not shown in the figures, with three ports respectively connected to one of the two portions of the first containment chamber 11, to the other of the two portions of the first containment chamber 11, and can be transferred to the needle 4 possibly by means of the needle holder component 3.

Figures from 6 to 10 show an advantageous, non-limiting embodiment of a syringe 100 according to the present invention, which can be used for example with the deformable cartridge 1 described above in order to perform an injection.

In a general embodiment, the syringe 100 comprises:
- a main body 101 provided with a receiving compartment 106 for receiving a deformable cartridge 1, wherein the main body 101 extends between a first end portion 102, where a first opening 103 is present, and an opposed second end portion 104, where a second opening 105 is present; and
- a crushing device 200 comprising a plunger 200 sliding with respect to the main body 101 between a retracted position and an advanced position and bearing a crushing head 201 sliding with respect to the main body 101 inside the receiving compartment 106 for deforming, in particular crushing, the deformable cartridge 1.

For the purpose of the present description, the expressions retracted position and advanced position refers to the main body 101 of the syringe 100.

According to an embodiment, the main body 101 of the syringe 100 is a tubular body, preferably flattened, which predominantly extends along a main axis X between the first end portion 102 and the second end portion 104.

The syringe 100 further comprises a support and fixing element 120 of the sliding plunger 200 to which the plunger 200 is slidably constrained.

According to a particularly advantageous embodiment, the syringe 100 comprises coupling means which allow the removable coupling of the support and fixing element 120 to the main body 101 and/or which allow the support and fixing element 120 to be coupled to the main body 101 so as to be able to change a mutual position between the support and fixing element 120 and the main body 101.

For example, if the aforesaid coupling means allow the removable coupling of the support and fixing element 120 to the main body 101, such coupling means may be conjugated reversible interlocking snap elements provided on the support and fixing element 120 and on the main body 101, respectively. In such a manner, the support and fixing element 120 can be repeatedly attached to and detached from the main body 101.

If the aforesaid coupling means allow the coupling of the support and fixing element 120 to the main body 101 so as to change a mutual position between the support and fixing element 120 and the main body 101 and said means allow rotating or slidably constraining the support and fixing element 120 to the main body 101. For example, such means comprise a cylindrical hinge which allows the mutual rotation between the support and fixing element 120 and the main body 101 about a rotation axis. For example, such a cylindrical hinge comprises a cylindrical pin 109 which projects from the main body 101 of the syringe 1 and further comprises a hole or opening provided on the support and fixing element 120 into which the pin 109 is inserted. It is worth noting that the arrangement of the pin and of the hole or opening can be reversed.

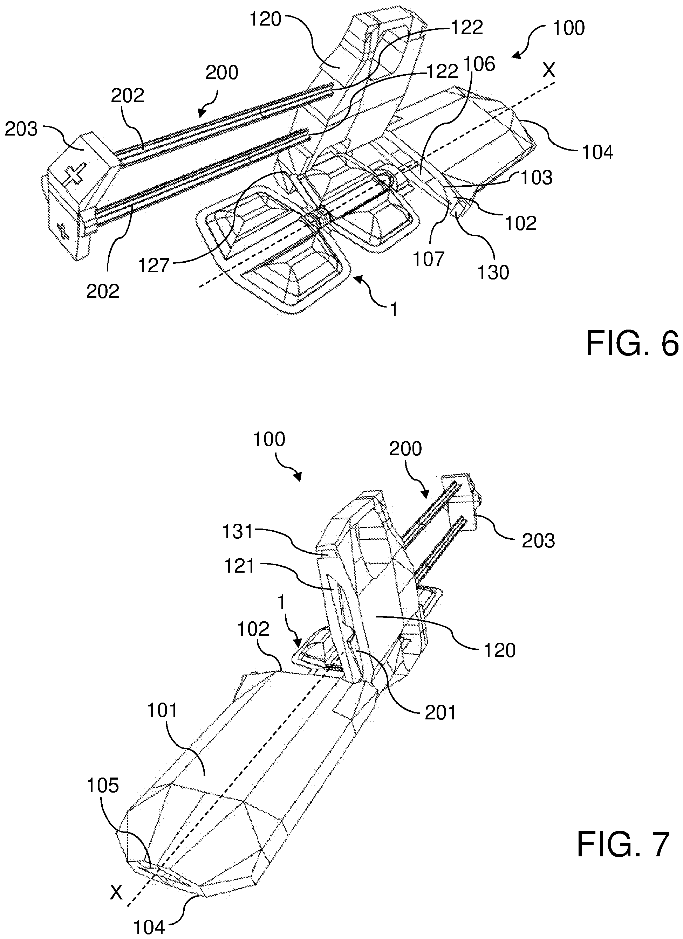

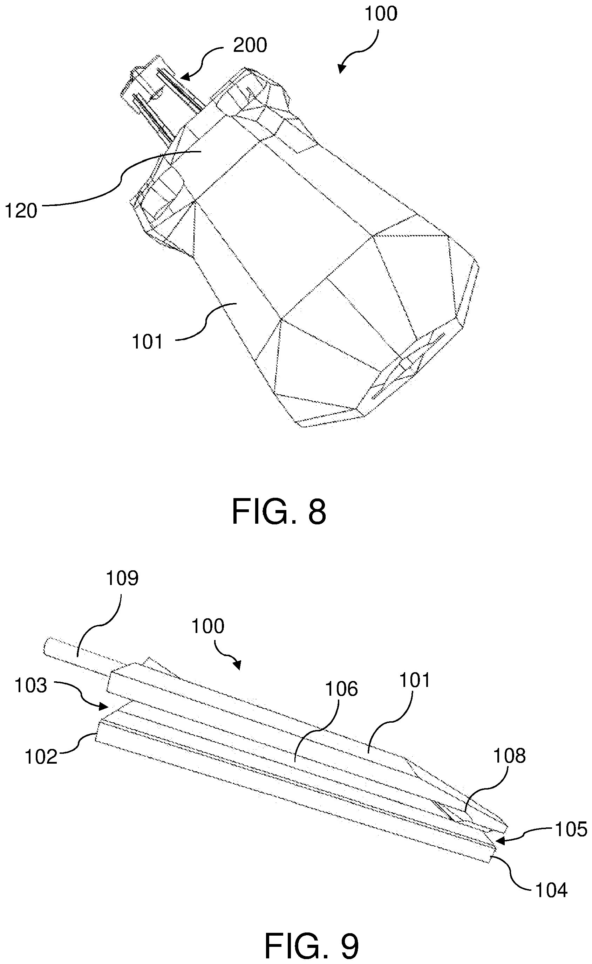

By virtue of the aforesaid coupling means, the syringe 100 may take at least two operating configurations. In one of such operating configurations, shown in figure 8, the support and fixing element 120 occludes the first opening 103. Such an operating configuration can therefore be referred to as the closing operating position. In the other operating configuration, shown in figures 6 and 7, the support and fixing element 120 allows access to the first opening 103, in order to insert the deformable cartridge 1 into the compartment 106 and/or to extract the deformable cartridge 1 from compartment 106. Such an operating configuration can therefore be referred to as the opening operating position.

According to an advantageous embodiment, the syringe 100 comprises locking means 130,131 which allow the syringe to be removed and locked in the closing operating configuration. Such locking means comprise, for example, an interlocking system having, for example, a tooth 130 on the main body 101 and a recess 131, preferably counter-shaped with respect to the tooth 130 and provided in the support and fixing element 120 into which the tooth 130 can be inserted. Obviously, the positions of the tooth 130 and of the recess 131 can be reversed.

According to an advantageous embodiment, a recess 204 is defined in the crushing head 201 which allows it to pass over the separation device 12,22, if provided in the deformable cartridge 1.

Conveniently, the recess 204 is shaped and sized to pass over the separation device 12,22 without touching it and/or without crushing it.

The crushing head 201 can be adapted and configured to move with respect to the deformable cartridge 1 according to an exclusive translation movement or according to a combined translation and rotation movement. In the latter case, the crushing head 201 could be or comprise at least one roll adapted and configured to slide on the deformable cartridge 1 by rotating on it. For example, the recess 204 in the roll is a continuous circular groove on a cylindrical wall of the roll.

As mentioned, the sliding plunger 200 carries the crushing head 201. The crushing head 201 can be inserted into the main body 101 through the first opening 103 and sliding inside the main body 101 together with the sliding plunger 200.

The sliding plunger 200 preferably comprises at least one stem 202, which has the crushing head 201 at one end and has a thrust element 203 at the other end. A user can apply a manual thrust force, e.g. by means of a thumb, onto such a thrust element 203 in order to obtain the sliding of the plunger 200 and thus of the crushing head 201, with respect to the main body 101 and with respect to the deformable cartridge 1. According to an advantageous embodiment, the sliding plunger 200 comprises two parallel stems 202, which are mutually spaced apart. This allows either limiting or preventing an unwanted deflection of the stems 202 during the sliding of the plunger 200 from the retracted position to the advanced position. This also makes the sliding movement more smooth and stable.

As mentioned above, the main body 101 of the syringe 100 has a receiving compartment 106 therein. Such a receiving compartment 106 can be accessed by means of the first opening 103 and is adapted to accommodate the deformable cartridge 1. According to an advantageous embodiment, the receiving compartment 106 comprises one or more guide grooves 107, e.g. two parallel and mutually spaced grooves, adapted and configured to allow an insert of the deformable cartridge 1 into the receiving compartment 106 for guided sliding along a sliding axis, e.g. along the main axis X. Such guiding grooves 107 are preferably adapted to receive respective peripheral edges of the cartridge body 10,20.

The second opening 105 can be crossed by the needle 4 during the insertion of the deformable cartridge 1 into the receiving compartment 106. Furthermore, the needle holder component 3 can preferably be locked in the second opening 105, e.g. by shape coupling or interference.

If the crushing head 201 is adapted and configured to be exclusively translated (i.e. free from rolls) with respect to the deformable cartridge 1, according to an advantageous embodiment such a crushing head 201 has an asymmetrical shape with respect to the main axis X, in order to achieve a preferential crushing on a side of the deformable cartridge 1. According to an advantageous embodiment, the crushing head 201 comprises a flat face and an opposite convex and bulging face. The recess 204 is preferably defined in the flat face. The flat face is configured to crush the deformable walls of the cartridge 1.

According to an advantageous embodiment, the receiving compartment 106 has a concave bottom 107, counter-shaped with respect to the crushing head 201, so as to be complementary thereto and follow the crushing of the deformable cartridge 1.

According to an advantageous embodiment, the support and fixing element 120 of the plunger 200 comprises a receiving seat 121 adapted to receive the crushing head 201 when the sliding plunger 200 is in the retracted position. Preferably, the support and fixing element 120 of the sliding plunger 200 comprises a through opening 122 communicating with the housing seat 121 which is crossed by at least one stem 202 of the sliding plunger 200. In the example shown in the figures, there are two through openings 122, each of which is crossed by a respective stem 202.

According to a particularly advantageous embodiment, the support and fixing element 120 of the plunger 200 comprises at least one pre-alignment element which allows the at least partial alignment of the deformable cartridge 1 with respect to the main body 101 before inserting the deformable cartridge 1 into the receiving compartment 106 and possibly also during the insertion of the cartridge itself. The aforesaid pre-alignment element comprises, for example, a guide groove 127.

In normal operation, the syringe 100 is initially in a starting configuration in which the sliding plunger 200 is in the retracted position in order to leave free the receiving compartment 106 adapted to house the deformable cartridge 1.

In such a configuration of the syringe 100, the support and fixing element 120 of the plunger 200 is detached from the main body 101 of the syringe 1 or is oriented or generally positioned so as to allow access to the first opening 103 and insertion of the deformable cartridge 1 into the receiving compartment 106 through it.

If the separation device 12,22 either is or comprises a connector, the cartridge body 10,20 is, for example, initially separated into two parts. Before the insertion into the syringe 1, the two parts 10,20 of the cartridge body are fixed together using connector 12,22.

The deformable cartridge 1 is then inserted and the needle 4 crosses the second opening 105 during the insertion. Once the insertion is complete, the support and fixing element 120 of the plunger 200 is positioned, e.g. rotated, so as to occlude the first opening 103. The configuration shown in figure 8 is thus achieved and at this point the syringe 1 is ready for use.

By acting on the plunger 200, the crushing head 201 crushes the deformable cartridge 1. For example, the crushing head 201 initially crushes the deformable walls of the second containment chamber 21. Once a given pressure is achieved inside the second containment chamber 21, the liquid component housed in the second containment chamber 21 passes through the fluidic communication duct 2 provided in the separation device 12,22 and enters into the first containment chamber 11. The crushing head 201 continues to advance until the second containment chamber 21 is completely emptied. By virtue of the recess 204, the crushing head 201 overcomes the separation device 12,22 passing over it without jamming. At this point, the operator can possibly suspend the advancement of the sliding plunger 200 and shake the syringe in order to facilitate the reconstitution of the solution for injection inside the first containment chamber 11. After passing through the separation device 12,22 the crushing head 201 continues its forward movement to crush the deformable walls of the first containment chamber 11. Once a given pressure is achieved inside the first containment chamber 11, the peelable partition provided between the first containment chamber 4 is peeled and possibly after the release of unwanted air the crushing head 201 is advanced further to inject the injectable solution.

After the injection, the syringe 100 can be opened and the cartridge 1 can be removed, e.g. to be introduced into a material recovery cycle. The syringe 100, on the other hand, is preferably reusable.

An injection kit comprising the syringe 100 and the deformable cartridge 1 is a further object of the present invention. In a variant embodiment, the injection kit comprises a syringe 100 and a plurality of deformable cartridges 1.

Innovatively, the syringe according to the present invention satisfies the requirements described above with reference to the syringes of the prior art. Indeed, a syringe as described above allows an easy and precise insertion of the deformable cartridge inside the syringe.

The system also has a lower cost for the end user and a benefit for the environment, because the syringe 100 is reusable.

It is apparent that a person skilled in the art may made changes to the syringe described above, all of which are contained within the scope of protection as defined in the following claims to satisfy contingent needs.

## Claims

1. A syringe (100) for the injection of an injectable solution contained in a deformable cartridge (1), wherein the syringe (100) comprises:
- a main body (101) provided with a receiving compartment (106) for receiving the deformable cartridge (1), wherein the main body extends between a first end portion (102), where a first opening (103) is present, and an opposed second end portion (104), where a second opening (105) is present;
- a crushing device (200) comprising a plunger (200) sliding with respect to the main body (101) between a retracted position and an advanced position and bearing a crushing head (201) sliding with respect to the main body (101) inside the receiving compartment (106) for deforming, in particular crushing, the deformable cartridge (1);
- a support and fixing element (120) of the sliding plunger (200) to which the plunger (200) is slidably constrained;
- coupling means for removably coupling the support and fixing element (120) to the main body (101) and/or which enable the support and fixing element (120) to be coupled to the main body (101) so as to change a mutual position between the support and fixing element (120) and the main body (101);
**characterized in that** said coupling means allow coupling the support and fixing element (120) to the main body (101) so as to change a mutual position between the support and fixing element (120) and the main body (101) and wherein said means allow rotating or slidably constraining the support and fixing element (120) to the main body (101).

2. A syringe (1) according to claim 1, wherein said coupling means allow removably coupling the support and fixing element (120) to the main body (101) and comprise conjugated reversible interlocking snap elements provided on the support and fixing element (120) and on the main body (101), respectively.

3. A syringe (1) according to claim 1, wherein said coupling means comprise a cylindrical hinge which allows rotating between them the support and fixing element (120) and the main body (101) about a rotation axis.

4. A syringe (1) according to any one of the preceding claims, wherein the syringe (100) can take at least two operating configurations, in one of which the support and fixing element (120) occludes the first opening (103) and in the other of which the support and fixing element (120) allows access to the first opening (103), in order to be able to insert the deformable cartridge (1) into the compartment (106) and/or to be able to extract the deformable cartridge (1) from the compartment (106).

5. A syringe (1) according to any one of the preceding claims, wherein the sliding plunger (200) comprises two parallel stems (202) spaced apart from each other.

6. A syringe (1) according to any one of the preceding claims, wherein the receiving compartment (106) comprises one or more guide grooves (107) adapted and configured to allow insertion of the deformable cartridge (1) into the receiving compartment (106) for the guided sliding along a sliding axis.

7. A syringe (1) according to any one of the preceding claims, wherein the support and fixing element (120) of the plunger (200) comprises a receiving seat (121) adapted to receive the crushing head (201) when the sliding plunger (200) is in the retracted position.

8. A syringe (1) according to any one of the preceding claims, wherein the support and fixing element (120) of the plunger (200) comprises at least one pre-alignment element (127) which allows at least partially aligning the deformable cartridge (1) with respect to the main body (101) before inserting the deformable cartridge (1) into the receiving compartment (106).

9. An injection kit for the injection of an injectable solution, comprising a syringe (100) according to any one of the preceding claims and at least one deformable cartridge (1) containing an injectable solution and wherein said cartridge is inserted or insertable into said syringe.

## Patentansprüche

1. Spritze (100) zum Einspritzen einer injizierbaren Lösung, die in einer verformbaren Kartusche (1) enthalten ist, wobei die Spritze (100) Folgendes umfasst:
- einen Hauptkörper (101), der mit einem Aufnahmefach (106) zum Aufnehmen der verformbaren Kartusche (1) versehen ist, wobei sich der Hauptkörper zwischen einem ersten Endabschnitt (102), an dem eine erste Öffnung (103) vorhanden ist, und einem gegenüberliegenden zweiten Endabschnitt (104), an dem eine zweite Öffnung (105) vorhanden ist, erstreckt;
- eine Zerkleinerungsvorrichtung (200), die einen Kolben (200) umfasst, der in Bezug auf den Hauptkörper (101) zwischen einer zurückgezogenen Position und einer vorgeschobenen Position gleitet und einen Zerkleinerungskopf (201) trägt, der im Inneren des Aufnahmefachs (106) in Bezug auf den Hauptkörper (101) gleitet, um die verformbare Kartusche (1) zu verformen, insbesondere zu zerkleinern;
- ein Stütz- und Befestigungselement (120) des gleitenden Kolbens (200), an dem der Kolben (200) gleitend gehalten wird;
- Kopplungsmittel zum lösbaren Koppeln des Stütz- und Befestigungselements (120) mit dem Hauptkörper (101) und/oder die eine Kopplung des Stütz- und Befestigungselements (120) mit dem Hauptkörper (101) ermöglichen, um so die Position des Stütz- und Befestigungselements (120) und des Hauptkörpers (101) zueinander zu ändern;
**dadurch gekennzeichnet, dass** die Kopplungsmittel ein Koppeln des Stütz- und Befestigungselements (120) mit dem Hauptkörper (101) ermöglichen, um so die Position des Stütz- und Befestigungselements (120) und des Hauptkörper (101) zueinander zu ändern, und wobei die Mittel ein drehbares oder gleitendes Halten des Stütz- und Befestigungselements (120) am Hauptkörper (101) ermöglichen.

2. Spritze (1) nach Anspruch 1, wobei die Kopplungsmittel ein lösbares Koppeln des Stütz- und Befestigungselements (120) mit dem Hauptkörper (101) ermöglichen und konjugierte reversible ineinandergreifende Schnappelemente umfassen, die am Stütz- und Befestigungselement (120) bzw. am Hauptkörper (101) vorgesehen sind.

3. Spritze (1) nach Anspruch 1, wobei die Kopplungsmittel ein zylindrisches Scharnier umfassen, das eine Drehung zwischen dem Stütz- und Befestigungselement (120) und dem Hauptkörper (101) um eine Drehachse ermöglicht.

4. Spritze (1) nach einem der vorhergehenden Ansprüche, wobei die Spritze (100) mindestens zwei Betriebskonfigurationen annehmen kann, wobei in einer davon das Stütz- und Befestigungselement (120) die erste Öffnung (103) verschließt und in der anderen das Stütz- und Befestigungselement (120) den Zugang zur ersten Öffnung (103) ermöglicht, um die verformbare Kartusche (1) in das Fach (106) einführen und/oder die verformbare Kartusche (1) aus dem Fach (106) entnehmen zu können.

5. Spritze (1) nach einem der vorhergehenden Ansprüche, wobei der gleitende Kolben (200) zwei parallele, voneinander beabstandete Schäfte (202) umfasst.

6. Spritze (1) nach einem der vorhergehenden Ansprüche, wobei das Aufnahmefach (106) eine oder mehrere Führungsnuten (107) umfasst, die geeignet und konfiguriert sind, um das Einführen der verformbaren Kartusche (1) in das Aufnahmefach (106) zum geführten Gleiten entlang einer Gleitachse zu ermöglichen.

7. Spritze (1) nach einem der vorhergehenden Ansprüche, wobei das Stütz- und Befestigungselement (120) des Kolbens (200) einen Aufnahmesitz (121) umfasst, der dazu geeignet ist, den Zerkleinerungskopf (201) aufzunehmen, wenn sich der gleitende Kolben (200) in der zurückgezogenen Position befindet.

8. Spritze (1) nach einem der vorhergehenden Ansprüche, wobei das Stütz- und Befestigungselement (120) des Kolbens (200) mindestens ein Vorausrichtungselement (127) umfasst, das eine zumindest teilweise Ausrichtung der verformbaren Kartusche (1) gegenüber dem Hauptkörper (101) ermöglicht, bevor die verformbare Kartusche (1) in das Aufnahmefach (106) eingeführt wird.

9. Injektionskit zum Einspritzen einer injizierbaren Lösung, umfassend eine Spritze (100) nach einem der vorhergehenden Ansprüche und mindestens eine verformbare Kartusche (1), die eine injizierbare Lösung enthält, und wobei die Kartusche in die Spritze eingeführt oder einführbar ist.

## Revendications

1. Seringue (100) pour l'injection d'une solution injectable contenue dans une cartouche déformable (1), la seringue (100) comprenant :
- un corps principal (101) pourvu d'un compartiment de réception (106) pour recevoir la cartouche déformable (1), dans laquelle le corps principal s'étend entre une première partie d'extrémité (102), où une première ouverture (103) est présente, et une seconde partie d'extrémité (104) opposée, où une seconde ouverture (105) est présente ;
- un dispositif d'écrasement (200) comprenant un piston (200) coulissant par rapport au corps principal (101) entre une position rétractée et une position avancée et portant une tête d'écrasement (201) coulissant par rapport au corps principal (101) à l'intérieur du compartiment de réception (106) pour déformer, en particulier écraser, la cartouche déformable (1) ;
- un élément de soutien et de fixation (120) du piston (200) coulissant auquel le piston (200) est contraint de manière coulissante ;
- des moyens d'accouplement pour accoupler de manière amovible l'élément de soutien et de fixation (120) au corps principal (101) et/ou qui permettent à l'élément de soutien et de fixation (120) d'être accouplé au corps principal (101) de manière à changer une position mutuelle entre l'élément de soutien et de fixation (120) et le corps principal (101) ;
**caractérisée en ce que** lesdits moyens d'accouplement permettent d'accoupler l'élément de soutien et de fixation (120) au corps principal (101) de manière à changer une position mutuelle entre l'élément de soutien et de fixation (120) et le corps principal (101) et dans laquelle lesdits moyens permettent de faire tourner ou de contraindre de manière coulissante l'élément de soutien et de fixation (120) par rapport au corps principal (101).

2. Seringue (1) selon la revendication 1, dans laquelle lesdits moyens d'accouplement permettent d'accoupler de manière amovible l'élément de soutien et de fixation (120) au corps principal (101) et comprennent des éléments d'encliquetage interpénétrés réversibles conjugués respectivement prévus sur l'élément de soutien et de fixation (120) et sur le corps principal (101).

3. Seringue (1) selon la revendication 1, dans laquelle lesdits moyens d'accouplement comprennent une charnière cylindrique qui permet de faire tourner entre eux l'élément de soutien et de fixation (120) et le corps principal (101) autour d'un axe de rotation.

4. Seringue (1) selon l'une quelconque des revendications précédentes, la seringue (100) pouvant prendre au moins deux configurations de fonctionnement, dans l'une l'élément de soutien et de fixation (120) obstruant la première ouverture (103) et dans l'autre l'élément de soutien et de fixation (120) permettant d'accéder à la première ouverture (103), afin de pouvoir insérer la cartouche déformable (1) dans le compartiment (106) et/ou de pouvoir extraire la cartouche déformable (1) du compartiment (106).

5. Seringue (1) selon l'une quelconque des revendications précédentes, dans laquelle le piston (200) coulissant comprend deux tiges parallèles (202) espacées l'une de l'autre.

6. Seringue (1) selon l'une quelconque des revendications précédentes, dans laquelle le compartiment de réception (106) comprend une ou plusieurs rainures de guidage (107) adaptées et conçues pour permettre l'insertion de la cartouche déformable (1) dans le compartiment de réception (106) pour le coulissement guidé le long d'un axe de coulissement.

7. Seringue (1) selon l'une quelconque des revendications précédentes, dans laquelle l'élément de soutien et de fixation (120) du piston (200) comprend un siège de réception (121) adapté pour recevoir la tête d'écrasement (201) lorsque le piston (200) coulissant est en position rétractée.

8. Seringue (1) selon l'une quelconque des revendications précédentes, dans laquelle l'élément de soutien et de fixation (120) du piston (200) comprend au moins un élément de pré-alignement (127) qui permet d'aligner au moins partiellement la cartouche déformable (1) par rapport au corps principal (101) avant d'insérer la cartouche déformable (1) dans le compartiment de réception (106).

9. Kit d'injection pour l'injection d'une solution injectable, comprenant une seringue (100) selon l'une quelconque des revendications précédentes et au moins une cartouche déformable (1) contenant une solution injectable et dans lequel ladite cartouche est insérée ou peut être insérée dans ladite seringue.
